# EUROPEAN PATENT SPECIFICATION

(11) **EP 1 499 735 B1**
(45) Date of publication and mention of the grant of the patent: **05.08.2009**
(21) Application number: 03732299.7
(22) Date of filing: 29.04.2003
(51) Int. Cl.: C12N 15/85, C12N 15/11

(54) **OPTIMIZATION OF TRANSGENE EXPRESSION IN MAMMALIAN CELLS**
OPTIMIERUNG VON TRANSGENEXPRESSION IN SÄUGETIERZELLEN
OPTIMISATION DE L'EXPRESSION DE TRANSGENES DANS DES CELLULES MAMMIFERES

(30) Priority: 30.04.2002 EP 02291091
(43) Date of publication of application: 26.01.2005
(73) Proprietor: Centre National de La Recherche Scientifique-CNRS, 75794 Paris Cedex 16 (FR); Biovectys, 75116 Paris (FR)
(72) Inventor: MALLET, Jacques, F-75013 Paris (FR); BRUN, Sophie, F-75006 Paris (FR); DUFOUR, Noelle, F-91540 Mennecy (FR); FAUCON-BIGUET, Nicole, F-75014 Paris (FR)
(74) Representative: Becker, Philippe
(86) International application number: PCT/EP2003/004457
(87) International publication number: WO 2003/093485

(56) References cited:
- ARONOV S ET AL: "Identification of 3'UTR region implicated in tau mRNA stabilization in neuronal cells." JOURNAL OF MOLECULAR NEUROSCIENCE, (1999 APR) 12 (2) 131-45., XP001119458 cited in the application
- SADOT E ET AL: "Complete sequence of 3'-untranslated region of Tau from rat central nervous system. Implications for mRNA heterogeneity." JOURNAL OF MOLECULAR BIOLOGY, (1994 AUG 12) 241 (2) 325-31., XP002219370
- SCHAMBACH A ET AL: "Context dependence of different modules for posttranscriptional enhancement of gene expression from retroviral vectors." MOLECULAR THERAPY, (2000 NOV) 2 (5) 435-45., XP002219371 cited in the application

### SEQUENCE LISTING

<110> CNRS and BIOVECTYS
<120> Optimization of transgene expression in mammalian cells.
<130> B0119WO
<140>
   <141>
<160> 4
<170> PatentIn Ver. 2.1
<210> 1
   <211> 609
   <212> DNA
   <213> Artificial Sequence
<220>
   <223> Description of Artificial Sequence: Primer
<400> 1
   aattcccgat aatcaacctc tggattacaa aatttgtgaa agattgactg gtattcttaa 60
   ctatgttgct ccttttacgc tatgtggata cgctgcttta atgcctttgt atcatgctat 120
   tgcttcccgt atggctttca ttttctcctc cttgtataaa tcctggttgc tgtctcttta 180
   tgaggagttg tggcccgttg tcaggcaacg tggcgtggtg tgcactgtgt ttgctgacgc 240
   aacccccact ggttggggca ttgccaccac ctgtcagctc ctttccggga ctttcgcttt 300
   ccccctccct attgccacgg cggaactcat cgccgcctgc cttgcccgct gctggacagg 360
   ggctcggctg ttgggcactg acaattccgt ggtgttgtcg gggaagctga cgtcctttcc 420
   atggctgctc gcctgtgttg ccacctggat tctgcgcggg acgtccttct gctacgtccc 480
   ttcggccctc aatccagcgg accttccttc ccgcggcctg ctgccggctc tgcggcctct 540
   tccgcgtctt cgccttcgcc ctcagacgag tcggatctcc ctttgggccg cctccccgca 600
   tcgggaatt                                                        609
<210> 2
   <211> 95
   <212> DNA
   <213> Artificial Sequence
<220>
   <223> Description of Artificial Sequence: APP 5'UTR region.
<400> 2
   cgggagacgg cggcggtggc ggcgcgggca gagcaaggac gcggcggatc ccactcgcac 60
   agcagcgcac tcggtgcccc gcgcagggtc ggtac                            95
<210> 3
   <211> 237
   <212> DNA
   <213> Artificial Sequence
<220>
   <223> Description of Artificial Sequence: tau 3'UTR region.
<400> 3
   cgggccatcg tggatgggag tccgtgtgtg cctggagata accctggaca cctctgcttt 60
   tttttttttt actttagcgg ttgcctccta ggcctgactc cttcccatgt tgaactggag 120
   gcagccacgt tacgtgtcaa tgtcctggca tcagtatgaa cagtcagtag tcccagggca 180
   gggccacact tctcccatct tctgcttcca ccccagcttg tgattgctag cctccca    237
<210> 4
   <211> 91
   <212> DNA
   <213> Artificial Sequence
<220>
   <223> Description of Artificial Sequence: TH 3'UTR region.
<400> 4
   acccacaggt gccaggggcc tttcccaaag tctccatccc cttctccaac ctttcctggc 60
   ccagaggctt tcccatgtgt gtggctgggc c                                91

## Description

The present invention relates to vectors, compositions and methods for delivering transgenes into mammalian cells. The invention also relates to genetic constructs and recombinant cells suitable to produce such vectors. The invention more particularly relates to a vector suitable for transgene delivery into mammalian cells, wherein said vector comprises a chimeric genetic construct comprising a transgene operably linked to at least two distinct posttranscriptional regulatory elements functional in mammalian cells. This invention can be used in experimental, research, therapeutic or prophylactic areas.

The development of technologies for delivery of foreign genes to the central nervous system is opening the possibility of using a variety of promising treatments for human diseases, especially human neurodegenerative diseases. Gene delivery vectors need to fulfil several criteria of efficacy and safety before they can be used in humans. Successful clinical application requires effective transgene expression with a minimum of vector-associated toxicity. Viral vectors are the most widely used delivery systems for gene transfer to the CNS. Replication-defective recombinant adenoviruses, adeno-associated viruses and lentiviruses are attractive vectors as they can infect nondividing neurons and glial cells with high efficiency (Le Gal La Salle *et al*, 1993; Kaplitt *et al*, 1994; Naldini *et al*, 1996). However, their use is hindered by vector-associated toxicity and host immune response. Both these phenomena are dose dependent and compromise transduced cell viability (Thomas *et al*, 2001). It would thus be advantageous to minimise the vector load, and thus toxicity, to favour persistence of the transgene. One way of reducing viral doses while maintaining high levels of expression is to improve the expression per molecule of vector.

Previous efforts at enhancing transgene expression have mostly been directed at boosting transcription. However, other steps affect the level of transgene expression, including posttranscriptional events concerning the recombinant mRNA. mRNA splicing is required for efficient production of a variety of mRNAs, and previous attempts at posttranscriptional enhancement have primarily involved the addition of intron sequences at the 5'-or 3'-end of the RNA of interest (Choi *et al*, 1991). In some cases, expression is entirely dependent on the presence of an intron (β-globin, Buchman and Berg, 1988). Although the exact mechanism of this effect has not been elucidated, it is thought that the intron, or the process of splicing itself, may promote 3'-end formation (Antoniou *et al*, 1998; Nesic *et al*, 1993; Huang and Gorman, 1990), enhance mRNA stability in the nucleus and/or improve mRNA export to the cytoplasm (Ryu and Mertz, 1989). The posttranscriptional regulatory elements of the hepatitis B virus (HPRE), a cis-acting RNA sequence required for the cytoplasmic accumulation of viral RNAs, have the same effect as an intron on β-globin expression (Huang and Yen, 1995). Another regulatory element (WPRE), similar in function to the HPRE, has been described in the woodchuck hepatitis virus (Donello *et al*, 1998). This element has the most uniform and generally greatest effect in enhancing transgene expression. WPRE substantially increases expression of transgenes in transfected (Loeb *et al*, 1999) and infected cultured cells (Loeb *et al*, 1999; Zufferey *et al*, 1999; Schambach *et al*, 2000; Ramezani *et al*, 2000) when incorporated into the 3' untranslated region. The mechanism of WPRE enhancement, is not well understood, but is thought to be posttranscriptional: possibly, WPRE functions by stimulating various steps of RNA processing, including polyadenylation and RNA export (Huang *et al*, 1999; Loeb *et al*, 2000).

In recent years, the functions of the flanking regions of eukaryotic mRNAs have come under increasing scrutiny as many contain a number of signal elements that contribute to mRNA stability or efficiency of translation. However, such elements have not been tested in plasmid or viral vectors. One hypothesis of the authors of the invention was that they would be useful for gene transfer by decreasing the vector load needed to obtain substantial expression in a particular target cell or organ.

### BRIEF DESCRIPTION OF THE INVENTION

The present invention now provides compositions and constructs (e.g., chimeric genes, vectors, cells, etc.) allowing improved gene expression into mammalian cells, *in vitro, ex vivo* or *in vivo*. The invention stems from the discovery that highly increased gene expression levels can be obtained in mammalian cells, particularly in neural cells, by providing an appropriate combination of regulatory elements. In particular, the invention shows that combinations of several posttranscriptional regulatory elements in a chimeric genetic construct or vector unexpectedly lead to high levels of gene expression in mammalian cells, up to a 26 fold-increase as compared to control experiments. Furthermore, the present application shows that such particular genetic constructs or chimeric genes are fully active in various backbones, including in viral vectors, thereby conferring additional advantages in terms of efficiency of gene delivery and expression.

A first aspect of the present invention resides in a vector suitable for transgene delivery into mammalian cells, particularly in neural cells, wherein said vector comprises a chimeric genetic construct comprising a transgene operably linked to at least two distinct posttranscriptional regulatory elements functional in mammalian cells. The posttranscriptional regulatory elements are more preferably able to stabilize RNAs and/or to increase their processing. Particular constructs according to this invention comprise 2, 3 or 4 distinct posttranscriptional regulatory elements, which are operably linked together so as to cooperate in providing increased transgene expression. Preferred vectors are plasmids or viral vectors.

Another aspect of this invention resides in a chimeric genetic construct comprising a transgene operably linked to at least two distinct posttranscriptional regulatory elements functional in mammalian cells.

A further aspect of the invention relates to a recombinant cell comprising a chimeric genetic construct or a vector as mentioned above.

The present invention also relates to the use of a genetic construct, a vector or a recombinant cell as disclosed above, for the manufacture of a medicament to treat a human disease, particularly a neurodegenerative disease.

A further aspect of the invention relates to a composition comprising a chimeric genetic construct, a vector or a recombinant cell as disclosed above and a pharmaceutically acceptable excipient or carrier.

The invention also concerns a method of expressing a transgene in a mammalian cell *in vitro, ex vivo* or *in vivo*, the method comprising:
a) providing a chimeric genetic construct comprising said transgene operably linked to at least two distinct posttranscriptional regulatory elements, and
b) introducing said construct into mammalian cells, said introduction causing expression of said transgene in said mammalian cells.

The method is particularly suited for the expression of a transgene in neural cells, especially in glial cells and/or neuronal cells, but also in other cellular types such as fibroblasts, in culture or in a subject. The method may be used to express various transgenes, such as therapeutic products, enzymes, neurotransmitters, toxins, growth factors, etc.

The results obtained in the context of the present invention provide important information regarding the development of optimal gene transfer vectors for gene therapy as well as for the study of gene function.

### DETAILED DESCRIPTION OF THE INVENTION

The present invention is directed to genetic constructs and vectors suitable for efficient and improved transgene delivery and expression into mammalian cells, particularly into neural cells, typically of human origin. As indicated above, the invention is based, inter alia, on the use of particular combinations of regulatory elements which allow an optimized gene expression into human cells. The present invention thus describes the use of particularly advantageous posttranscriptional regulatory elements which, when operably combined and linked to a transgene, allow high-level transgene expression in cells. The applicants have indeed found that transgene expression in cells of various phenotypes, including neuronal cells (PC12, NGF-treated PC12, SKNSH for example), glial cells (C6, U-87MG for example) and other cellular types such as fibroblats, was substantially enhanced by combinations of appropriate posttranscriptional regulatory elements.

The present invention is thus directed to genetic constructs and vectors suitable for efficient and improved transgene delivery and expression into mammalian cells, wherein said vector comprises a chimeric genetic construct comprising a transgene operably linked to at least two distinct posttranscriptional regulatory elements functional in mammalian cells.

The expression "chimeric genetic construct", as used herein, means a nucleic acid construct artificially created (e.g., by assembling of various nucleic acids) using recombinant DNA techniques, such as ligation, cloning, digestion, hybridizations, etc. The chimeric genetic construct typically comprises a transgene encoding a biological product of interest, said transgene being operably linked to regulatory sequences such as, for instance, promoter, posttranscriptional regulatory elements, polyA region, targeting moiety, etc. Moreover, the chimeric genetic construct may further comprise a tag to facilitate purification or monitoring, such as a myc tag, a poly-histidine tag, etc. The chimeric genetic construct may be single-stranded or, more preferably double-stranded. It may be on the form of DNA or RNA. The chimeric genetic construct may be prepared by various techniques, including nucleic acid synthesis, amplification or isolation from libraries, chemical modification, etc.

The expression "operably linked", as used herein, means combined, fused or associated so as to functionaly cooperate or interact. The nucleic acids may be directly fused to each other, or separated by spacer regions that do not alter the properties of each compound of the chimeric genetic construct. The posttranscriptional regulatory element may be located upstream or dowstream from the transgene, in the same or inverse orientation. Furthermore, several copies of the postranscriptional regulatory elements may be used. The term "operably linked" generally indicates that the promoter regulates expression of the transgene and that the posttranscriptional regulatory elements affect the transductional expression of the transgene. Such spacer regions include cloning sites, cleavage sites, etc.

Various posttranscriptional regulatory elements coming from the flanking regions of eukaryotic mRNAs may be used, in combination, in the preparation of chimeric genetic constructs or vectors of this invention. The posttranscriptional regulatory element contain a number of signal elements that contribute to mRNA stability or efficiency of translation. As used in the present invention, they confer increased stability to mRNAs and lead to high levels of gene expression in mammalian cells, particularly in neural cells.

An object of the invention thus relates to a vector wherein at least one posttranscriptional regulatory element comprises all or a portion of a UTR region of a eukaryotic mRNA.

The inventors of the present invention tested various elements and sequences from eukaryotic mRNAs that could enhance transgene expression in mammalian cells: i) WPRE, ii) a fragment of the 3'UTR of rat tau mRNA, iii) a fragment of the 3'UTR of rat tyrosine hydroxylase (TH) mRNA, and vi) a fragment of the 5'UTR of human Alzheimer Amyloid Precursor (APP) mRNA. The inventors tested combinations of these posttranscriptional regulatory elements and unexpectedly found that they could cooperate or synergize to provide positive effects on transgene expression.

Of the several elements which were tested, WPRE gave the highest level of expression. Further enhancements are observed when WPRE is combined with sequences corresponding to the 5' or 3' untranslated regions (UTR) of eukaryotic mRNAs (tau 3'UTR, TH 3'UTR and APP 5'UTR).

As explained above, WPRE is a cis-acting RNA sequence required for the cytoplasmic accumulation of RNAs. The authors of the present invention have discovered that this element has also the most uniform and generally greatest effect in enhancing transgene expression in mammalian neural cells. WPRE substantially increases expression of transgenes in transfected and infected cultured cells when incorporated into the 3' untranslated region. The mechanism of WPRE is posttranscriptional. It has been demonstrated that WPRE functions by stimulating various steps of RNA processing, including polyadenylation and RNA export.

The invention thus comprises a vector as described above, wherein at least one posttranscriptional regulatory element comprises a WPRE element.

A preferred aspect of the invention relates to a vector as described above, wherein said WPRE element comprises all or a functional fragment of SEQ ID NO: 1.

The invention also includes a vector as described above, wherein at least one posttranscriptional regulatory element comprises all or a portion of a UTR region of a eukaryotic mRNA. The UTR region may be selected from a fragment of the WPRE, of the 5'UTR of human Alzheimer Amyloid Precursor (APP) mRNA (APP5'UTR), of the 3'UTR of rat tau mRNA (tau 3'UTR), of the 3'UTR of rat tyrosine hydroxylase (TH 3'UTR) mRNA or of a functional portion thereof.

A particular object of the present invention relates to a vector as described above suitable for transgene delivery into mammalian cells, wherein at least one posttranscriptional regulatory element is an APP 5'UTR region. In a further preferred aspect, APP 5'UTR region comprises all or a functional fragment of the Smal-Nrul SEQ ID NO: 2 (nucleotides 50-144).

The invention shows that in glial cells, WPRE and APP 5'UTR synergistically increase expression about 10-fold and that, in fibroblasts, WPRE and APP 5'UTR synergistically increase expression about 6-fold. A particular vector of this invention thus comprises a chimeric genetic construct comprising a transgene operably linked to a WPRE element and to an APP 5' UTR region.

The present invention also relates to a vector as described above suitable for transgene delivery into mammalian cells, wherein at least one posttranscriptional regulatory element is a tau 3'UTR region. In a preferred aspect, tau 3'UTR region comprises all or a functional fragment (nucleotides 2519-2760) of SEQ ID NO: 3.

The invention shows that in neuronal cell lines, WPRE and both tau 3'UTR and APP 5'UTR have a synergistic effect on expression (up to about 26 times basal level). Another particular object of the present invention thus concerns a vector suitable for transgene delivery into mammalian cells, for example in neural cells, wherein said vector comprises a chimeric genetic construct comprising a transgene operably linked to a WPRE element, an APP5'UTR region and a tau3'UTR region.

The present invention also relates to a vector as described above suitable for transgene delivery into mammalian cells, wherein at least one posttranscriptional regulatory element is a TH 3'UTR region. In a preferred aspect, TH3'UTR region comprises all or a functional fragment of SEQ ID NO: 4 (part of pTH51).

The invention shows that WPRE, APP 5'UTR and both tau3'UTR and TH3'UTR have a synergistic effect on expression. A further particular object of the present invention then concerns a vector suitable for transgene delivery into neural cells, wherein said vector comprises a chimeric genetic construct comprising a transgene operably linked to a WPRE element, an APP5'UTR region, a tau3'UTR region and a TH3'UTR region.

Further enhancement of expression in mammalian cells might be possible by using additional regulatory elements, such as introns. The present invention thus also concerns a vector as described above further comprising an intron.

In a preferred embodiment of the invention, the vector or chimeric genetic construct as described above, further comprises a promoter controling transcription of the transgene in said mammalian cells. Differents kinds of promoters may be used in the context of the present invention. They may be strong or weak, tissue-specific or ubiquitous, regulated or constitutive. Any promoter may generally be used as long as it is transcriptionnally functional in the targeted cell populations. It may thus be a viral promoter, a cellular promoter (house-keeping promoters, for example), a bacterial promoter, etc. For example, viral promoters may be selected from CMV, SV40, LTR, TK, etc. Cellular promoters may be chosen from the promoters of the β-actin, PGK, apolipoprotein, albumin and Ubiquitin C genes, and bacterial promoters may be selected from pLac, pTrp, T7, pTAC, etc. Most preferred promoters are viral or cellular promoters, as well as various combinations thereof or synthetic promoters.

The invention also includes a vector as described above, wherein said vector further comprises a marker gene. The "marker gene", as used herein, may be any gene or nucleic acid sequence whose expression can be detected, observed or measured by any known technique (auxotrophy, fluorescence, luminescence, resistance, etc.). Various genes may thus be used as a marker gene in the context of the present invention, including, without limitation,. the hygromycin, neomycin or phleomycin resistance genes as well as a gene coding for luciferase, phosphatase alcaline, galactosidase, lactamase or green fluorescent protein (GFP), for instance.

A further object of the present invention relates to a vector as described above, wherein said vector further comprises a polyadenylation signal operably linked to said transgene and posttranscriptionnal regulatory elements. Preferred polyadenylation signals are the SV40 polyadenylation signal and the bovine growth hormone polyadenylation signal.

The vector according to the present invention may be of various types and origins, such as, a plasmid, a recombinant virus, a cosmid, an artificial chromosome, an episome, etc. Most preferred vectors are plasmids or viral vectors, in particular plasmids and recombinant viruses. Typical examples include plasmids, such as those derived from commercially available plasmids, in particular pUC, pcDNA, pBR, etc. Other preferred vectors are derived from viruses, such as replication-defective adenovirus (e.g., Ad5, Ad2), replication-defective adeno-associated virus (rAAVs) or replication-defective retrovirus, including replication-defective lentiviruses, for example (MLV, FLV, HIV, EIAV, etc.), baculoviruses or vaccinia viruses. Recombinant viruses may be prepared using known techniques (e.g., packaging cells, transient transfection, helper plasmids or viruses, etc.). The choice of the vector may be made by the skilled person depending on the target cell, population, tissue or organism. Preferred vectors are able to infect or transfect mammalian cells *in vitro, ex vivo* or *in vivo*.

As indicated above, the vectors of the present invention comprise a transgene. The transgene may be any nucleic acid coding for a biological product (RNA, polypeptides, etc.). The nucleic acid may be a cDNA, an rRNA, a tRNA, a gDNA, preferably a cDNA. Generally, this invention can be used to produce any polypeptide of interest, e.g., any polypeptide having biological or immune properties. Furthermore, the invention can be used to simultaneously express or target several distinct chimeric genetic constructs encoding distinct polypeptides in cells, to further expand the scope of activities or reconstitute complex molecules. The encoded polypeptide may be any polypeptide and is preferably selected from a cytokine (IL-2, TNF, IFN, etc.), a ligand, a receptor, an immunoglobulin, a growth factor, a neurotrophic factor (e.g., GDNF, CNTF, NT, PDGF, SCF, etc.), an enzyme or a portion thereof. The nucleic acid and amino acid sequences of these polypeptides are known per se and can be obtained from DNA libraries, commercial plasmids or by recombinant DNA technologies.

The compositions and methods according to the invention surprisingly enhance the capacity of vector delivery systems to produce therapeutic products in mammalian cells, particularly in the neural cells of the CNS. The experimental part of this application demonstrates an enhancement of transgene expression in particular in neural cells of both phenotypes, neuronal and glial and also in fibroblasts. The combination of posttranscriptional regulatory elements act synergistically to increase expression, resulting in up to 10- and 26-fold enhancements in glial and neuronal cell lines, respectively, the combination of WPRE and APP 5'UTR allowing a 6-fold increase expression in fibroblasts. Thus, combinations of these elements allow therapeutic effects to be obtained in a subject with substantially less vector, thereby decreasing both the side effects associated with viral injection and the number of copies of transgenes required per cell for therapeutic effects.

The invention indeed includes a recombinant cell comprising a chimeric genetic construct as described above. Preferred recombinant host cells are mammalian cells. These can be primary cells or established cell lines. Illustrative examples include fibroblasts, neuronal and glial cells as well as their progenitor or precursor cells.

The present invention also describes the use of a chimeric genetic construct, vector or recombinant cell as described above for the manufacture of a medicament for treatring a human disease, in particular a neurodegenerative disease. The neurodegenerative disease may be selected from Parkinson's disease, Alzheimer's disease, amyotrophic lateral sclerosis (ALS), Huntington's disease and retinal degenerative diseases.

A further object of the invention relates to a composition comprising a chimeric genetic construct, a vector or a recombinant cell as described above and a pharmaceutically acceptable excipient or carrier and to the use of said composition for treating a human disease as described above.

A particular object of the present invention relates to a viral-mediated delivery of the trophic factor GDNF to treat Parkinson disease. GDNF indeed reverses or prevents the structural and functional correlates of nigrostriatal degeneration.

The invention also provides a method of expressing a transgene in a mammalian cell *in vivo, in vitro* or *ex vivo*, the method comprising:
a) providing a chimeric genetic construct comprising said transgene operably linked to at least two distinct posttranscriptional regulatory elements or a vector as described above, and
b) introducing said construct or said vector into mammalian cells, said introduction causing expression of said transgene in said mammalian cells.

In the context of the methods described above, mammalian cells are preferably selected from glial (C6, U-87MG for example), neuronal cells (PC12, NGF-treated PC12, SKNSH for example) and other cellular types such as fibroblasts. The mammal is preferably a human or a rodent (for example a mouse or a rat). Although particularly suited for gene delivery and expression into neural cells, the invention may also be used in other tissues, organs or cell types, such as hematopoietic cells, fibroblasts, epithelial cells, muscle cells (smooth and skeletic). Particular organs include CNS, brain, retina, liver, skin, muscle, etc.

In the method as described above, the genetic construct (or vector) can be introduced into mammalian cells by any conventional method, such as by naked DNA technique, cationic lipid-mediated transfection, polymer-mediated transfection, peptide-mediated transfection, virus-mediated infection, physical or chemical agents or treatments, etc. In this regard, it should be noted that transient transfection is sufficient to express the relevant chimeric gene so that it is not necessary to create stable cell lines or to optimize the transfection conditions. The chimeric genetic construct or vector may be introduced into mammalian cells during step b) preferably by virus-mediated infection, e.g. stereotaxic injection into the CNS (striatum and/or substantia nigra), or by plasmid-mediated transfection, e.g. electroporation.

A particular method of expressing a transgene in glial cells according to the invention is the method comprising:
a) providing a chimeric genetic construct comprising said transgene operably linked to posttranscriptional regulatory elements comprising a WPRE element combined with a APP5'UTR or a portion thereof, and
b) introducing said construct into glial cells, said introduction causing expression of said transgene in said glial cells.

Another particular method of expressing a transgene in fibroblasts according to the invention is the method comprising:
a) providing a chimeric genetic construct comprising said transgene operably linked to posttranscriptional regulatory elements comprising a WPRE element combined with a APP5'UTR or a portion thereof, and
b) introducing said construct into fibroblasts, said introduction causing expression of said transgene in said fibroblasts.

A further particular method of expressing a transgene in neuronal cells according to the invention is the method comprising:
a) providing a chimeric genetic construct comprising said transgene operably linked to posttranscriptional regulatory elements comprising a WPRE element combined with a APP5'UTR and a tau3'UTR or a portion thereof, and
b) introducing said construct into neuronal cells, said introduction causing expression of said transgene in said neuronal cells.

Another method of expressing a transgene in neuronal cells according to the invention is the method comprising:
a) providing a chimeric genetic construct comprising said transgene operably linked to posttranscriptional regulatory elements comprising a WPRE element combined with a APP5'UTR, a tau3'UTR and a TH3'UTR or a portion thereof,
b) introducing said construct into neuronal cells, said introduction causing expression of said transgene in said neuronal cells.

The following examples are offered by way of illustration and not by way of limitation.

### DESCRIPTION OF THE FIGURES

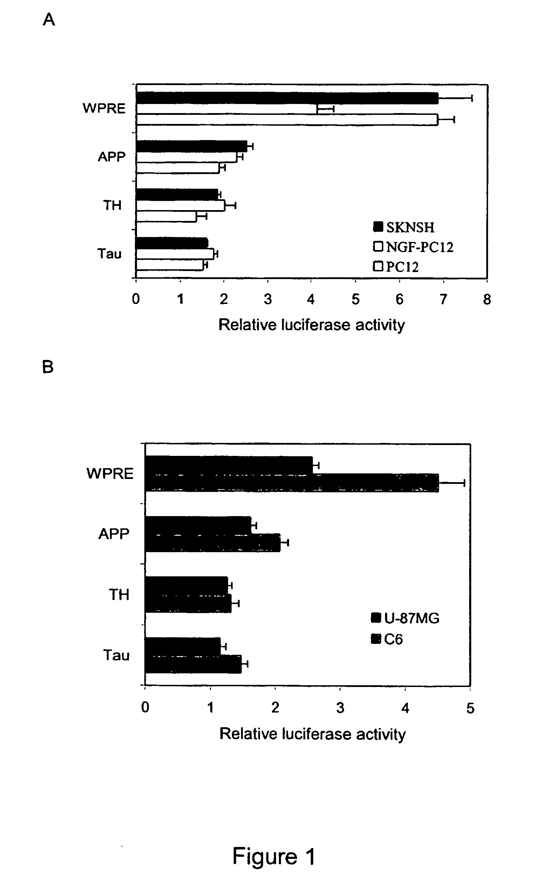

### Figure 1: Effects of tau 3'UTR, TH 3'UTR, APP 5'UTR and WPRE on luciferase expression in neuronal and glial cell lines.

A) Schematic representation of the constructs containing the posttranscriptional regulatory elements in the 3' or 5'end of the luciferase gene. These elements are WPRE, tau 3'UTR, TH 3'UTR and APP 5'UTR.
B) 0.58 pmoles (-2 µg) of the constructs pluc, pluc-tau, pluc-TH, pAPP-luc and pluc-WPRE were used to transfect (a) neuronal cell lines: PC12, NGF-treated PC12, SKNSH and (b) glial cell lines: C6, U-87MG. Luciferase activity obtained with each posttranscriptional regulatory element was normalized to renilla activity from a co-transfected internal control plasmid measured in the same extract. Normalized luciferase activities are expressed relative to that obtained by the construct lacking posttranscriptional regulatory elements (pluc), which is arbitrarily set at 1.0. Histograms represent mean ± s.e.m. of at least three independent experiments. In each experiment, triplicate transfections were carried out. Student's *t*-test was used to compare luciferase activities between constructs and pluc. *, Significant difference (p<0.05).

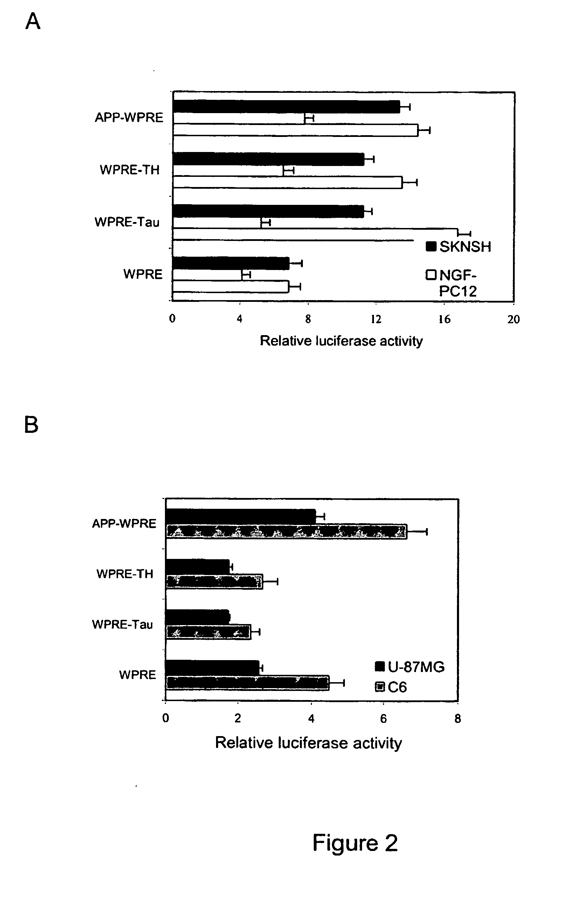

### Figure 2: Effects of WPRE paired with tau 3'UTR, TH 3'UTR or APP 5'UTR in neuronal and glial cell lines.

A) Schematic representation of the luciferase constructs containing WPRE and each of the eukaryotic sequences.
B) 58 fmoles (∼0.2 µg) of the constructs pluc-W, pluc-tau-W, pluc-TH-W and pAPP-luc-W were used to transfect (a) neuronal cell lines: PC12, NGF-treated PC12, SKNSH and (b) glial cell lines: C6, U-87MG. Luciferase activity obtained with each construct was normalized and expressed as described in Fig.1. *, Significantly different from the value obtained for WPRE (p<0.05, Student's *t-*test).

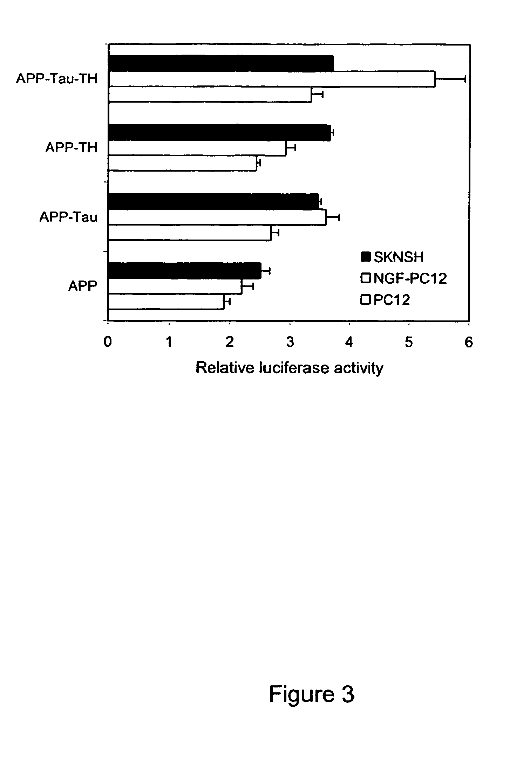

### Figure 3: Effects of combinations of tau 3'UTR, TH 3'UTR and APP 5'UTR in neuronal cell lines.

A) Schematic representation of the reporter gene constructs containing combinations of the eukaryotic sequences.
B) 58 fmoles (∼0.2 µg) of the constructs pAPP-luc-tau, pAPP-luc-TH and pAPP-luc-TH-tau were used to transfect neuronal cell lines: PC12, NGF-treated PC12 and SKNSH. Luciferase activity obtained with each construct was normalized and expressed as described in Fig.1. *, Significantly different from the value obtained for pAPP-luc; **, Significantly different from the value obtained for pAPP-luc-tau and for pAPP-luc-TH (p<0.05, Student's *t*-test). *ns*, not significant.

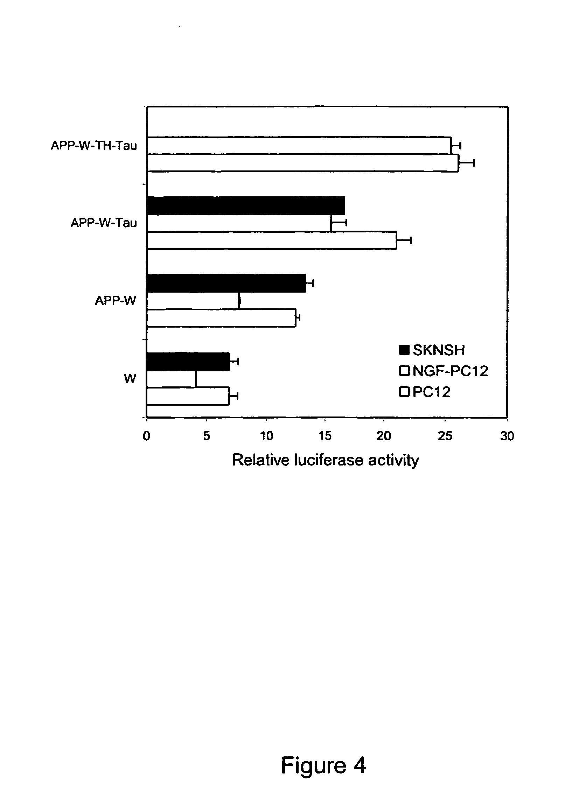

### Figure 4: Optimised combinations of posttranscriptional regulatory elements in neuronal cell lines.

A) Schematic representation of the luciferase constructs containing optimised combinations of WPRE with eukaryotic elements for expression in PC12, NGF-treated PC12 cells (pAPP-luc-WPRE-TH-tau) or in SKNSH cells (pAPP-luc-WPRE-tau).
B) SKNSH were transfected with 58 fmoles (∼0.2 µg) of pAPP-luc-WPRE-TH. PC12 and NGF-treated PC12 were transfected with ∼0.2 µg (58 fmoles) of pAPP-luc-WPRE-TH and pAPP-luc-WPRE-TH-tau. Luciferase activity obtained with each construct was normalized and expressed as described in Fig.1. *, Significantly different from the value obtained for pAPP-luc-WPRE (p<0.05, Student's *t*-test).

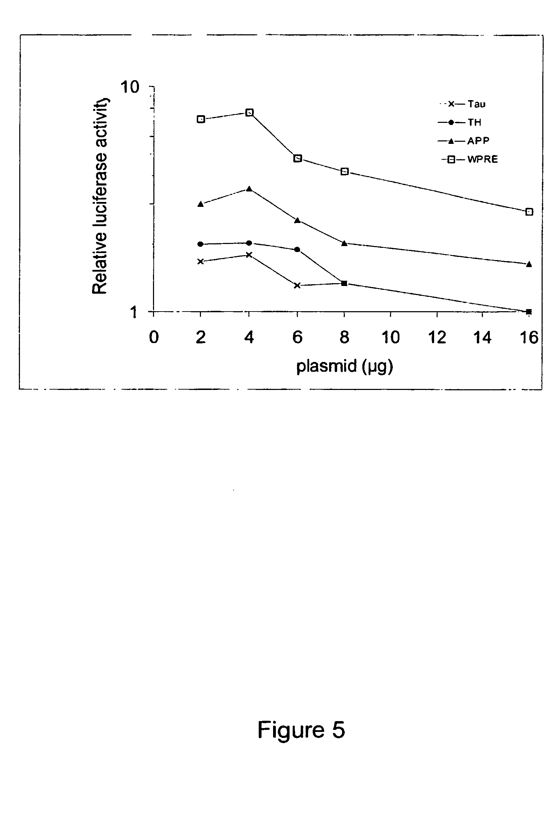

### Figure 5: Effects of tau 3'UTR, TH 3'UTR, APP 5'UTR and WPRE on luciferase expression depend on transfecting plasmid dose.

PC12 cells were transfected with various amount of pluc-tau, pluc-TH, pAPP-luc and pluc-WPRE (2-16 µg / 1.5x10⁶ cells). Luciferase activity obtained with each construct was normalized and expressed as described in Fig.1. Linear regression analysis indicated a correlation between amount of plasmid transfected and effects of tau 3'UTR, TH 3'UTR, TH 3'UTR and WPRE on luciferase expression, indicating a linear dose-dependency (correlation coefficient: r = 0.90 for tau 3'UTR; r = 0.94 for TH 3'UTR; r = 0.86 for APP 5'UTR and r = 0.89 for WPRE).

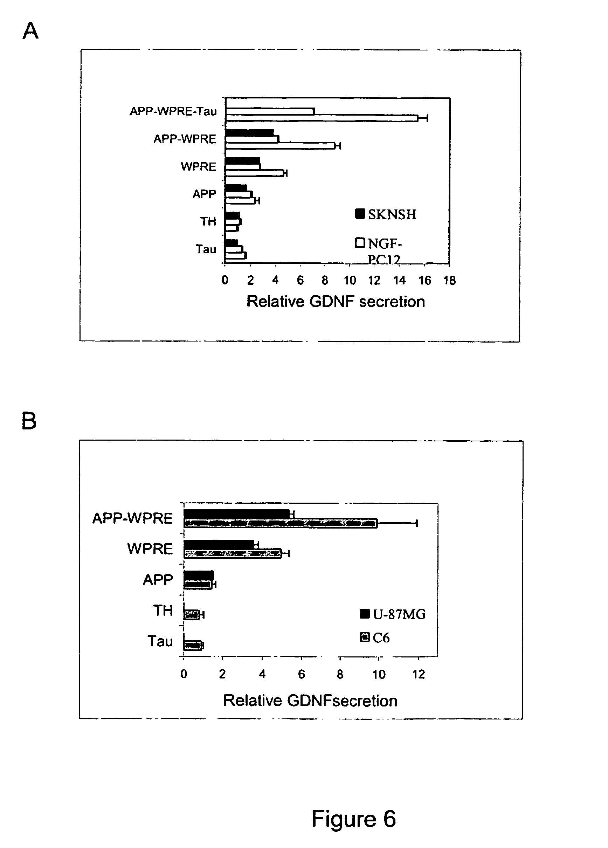

### Figure 6: Stimulation of GDNF secretion with combinations of posttranscriptional regulatory elements in neuronal and glial cell lines.

A) Schematic representation of the various constructs containing the posttranscriptional regulatory elements, alone or in combination, in the 3' or 5'ends of the gdnf gene.
B) PC12, NGF-treated PC12 and SKNSH were transfected with 58 fmoles (∼0.2 µg) of the constructs pgdnf, pgdnf-tau, pgdnf-TH, pAPP-gdnf, pgdnf-WPRE, pAPP-gdnf-WPRE and pAPP-gdnf-WPRE-tau. C6, U-87MG were transfected with 58 fmoles (∼0.2 µg) of pgdnf, pAPP-gdnf, pgdnf-WPRE and pAPP-gdnf-WPRE. Concentration of GDNF in the cell supernatant was normalized to renilla activity from a co-transfected internal control plasmid measured in the cell lysate. Normalized luciferase activities are expressed relative to that obtained for the construct lacking posttranscriptional regulatory elements (pgdnf), which is arbitrarily set at 1.0. Histograms represent mean ± s.e.m. of at least three independent experiments. In each experiment, triplicate transfections were carried out. *, Significantly higher than the value for pgdnf (p<0.05, Student's *t-*test). **, Significantly higher than the value obtained for pgdnf-WPRE. ***, Significantly higher than the value obtained for pAPP-gdnf-WPRE.

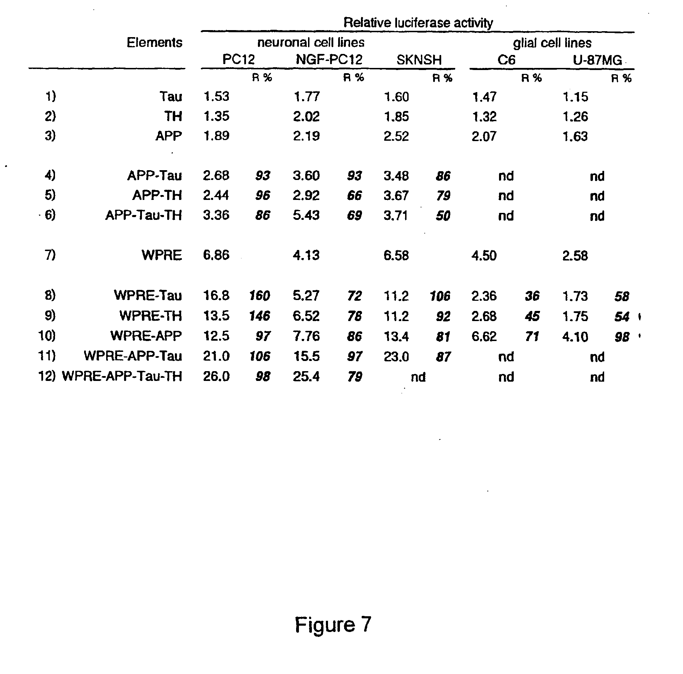

### Figure 7: Synergistic effects of WPRE, tau 3'UTR, TH 3'UTR and APP 5'UTR on luciferase expression. (cf. page 19)

Values of relative luciferase activities obtained for each construct containing tau 3'UTR, TH 3'UTR, APP 5'UTR and WPRE, alone or in combination, are presented (data are graphically represented in the degree to which the effects of each element is cumulative on expression when they were combined. This rate was calculated by dividing the relative luciferase activity obtained with the combination of elements by the product of the effect of each element. It is expressed as a percentage. A rate of 100% indicates that the elements have the same effects on enhancement of expression when they were combined as when they were alone in the UTR of the luciferase transcript. *nd*, not determined.

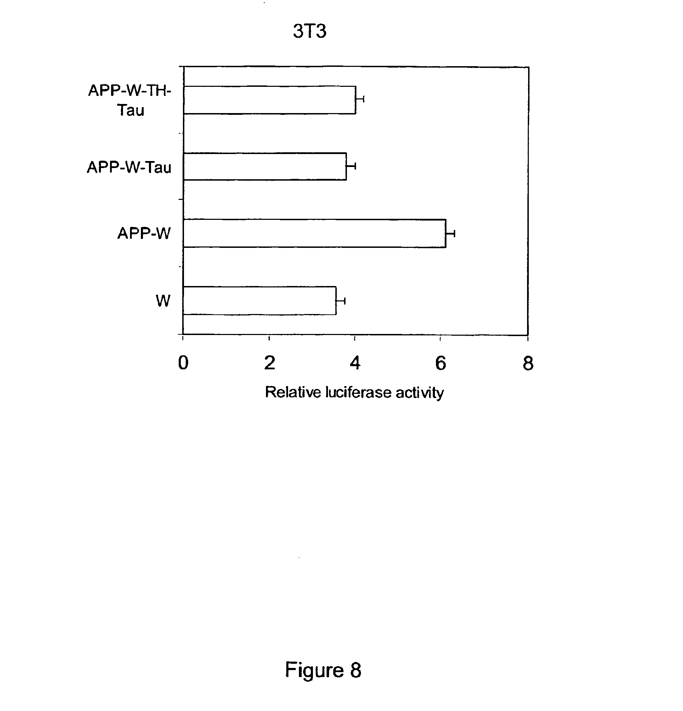

### Figure 8. The combination between WPRE and APP 5'UTR was also effective in the rat fibroblast cell line 3T3.

3T3 cells were transfected with 58 fmoles (∼0.2 µg) of pluc-WPRE pAPP-luc-WPRE, pAPP-luc-WPRE-TH and pAPP-luc-WPRE-TH-tau. Luciferase activity obtained with each construct was normalized and expressed as described in Figure 1.

### MATERIALS AND METHODS

### Plasmids constructions

A series of plasmid vectors expressing the firefly luciferase gene or the rat GDNF cDNA under the regulatory control of various posttranscriptional regulatory elements was constructed. Firefly luciferase plasmids were derived from pGL3-Control (Promega). GDNF plasmids are derived from pgdnf, which was generated by insertion of the entire rat GDNF cDNA (Bilang-Bleuel *et al,* 1997) into the BamHI site of pcDNA3 (Invitrogen) dowstream from the CMV promoter. The 241-bp fragment containing nucleotides 2519-2760 of the rat tau 3'UTR was provided by I. Ginzburg (Weizmann Institute of Science, Israel; Aronov *et al*, 1999). The fragment containing the first 90 bp of the rat TH 3'UTR was derived from pTH51 (fragment Kpnl-Apal; Grima *et al*, 1985). The 600-bp WPRE was provided by Dr D.Trono (Department of Genetics and Microbiology, Switzerland). These elements (WPRE, sequences from tau 3'UTR and TH 3'UTR) and combinations thereof were inserted into the luciferase and gdnf genes at a position corresponding to the 3' end of the mRNA by blunt-end ligation into the Xbal site and Apal site, respectively. Two complementary oligonucleotides containing the Smal-Nrul sequence of the human APP 5'UTR (nucleotides 50-144; Rogers *et al*, 1999) flanked by Kpnl restriction sites, were annealed. The resulting double-stranded oligonucleotide was inserted into the HindIII site of pGL3-Control vector, downstream from the SV40 promoter, and into the Kpnl site of pgdnf, downstream from the CMV promoter. All plasmid DNAs were prepared using the Jetstar plasmid purification system (Genomed). To simplify the experimental part of the text and the plasmid nomenclature, sequences from tau 3'UTR, TH 3'UTR and APP 5'UTR are designated as tau 3'UTR, TH 3'UTR and APP 5'UTR in the text and tau, TH and APP in plasmid constructions, respectively.

### Cell cultures

PC12 cells (rat pheochromocytoma) were grown in RPMI-1640 medium (Sigma) supplemented with 10% horse serum (Sigma) and 5% foetal calf serum (Sigma) and maintained at 37°C in 5% CO2. For NGF treatment, 1.25x10⁵cells were plated on collagen-coated 12-well plates and grown in DMEM (Sigma) supplemented with 1% foetal calf serum and 50 ng/ml of NGF (Sigma). NGF was added every 2 days for six or eight days. SKNSH (human neuroblastoma), C6 (rat astrocytoma) and U-87MG (human astrocytoma) were grown in DMEM supplemented with 10% foetal calf serum and 2mM of L-glutamine.

### Transient transfections

1.5-2.0 x 10⁶ cells (PC12, SKNSH, C6 and U-87MG) were re-suspended in 0.2 ml of serum-free medium and transfected by electroporation with 580 or 58 fmoles (∼ 2 or 0.2 µg) of one of the reporter plasmids and 0.02µg of a Renilla luciferase plasmid (pRL-SV40; Promega) as an internal control to correct for differences in transfection efficiency. A Biorad gene pulser at 190 V (PC12), 150 V (SKNSH) and 250 V (C6 and U-87MG), 960 µF for 50ms was used for electroporation. After electroporation, cells were placed in serum-containing medium. NGF-treated PC12 transfections were performed using the Lipofectamine Plus Reagent (Gibco Life Technologies). NGF-treated PC12 cells were transfected with mixtures of 330 or 33 fmoles (∼ 1.1 or 0.11µg) of one of the reporter plasmids, 0.11µg of pRL-SV40, and a carrier DNA (pBluescript, Stratagene) to give a total of 0.5µg of DNA, and 5µl of both Plus Reagent and Lipofectamine. Cells were harvested 48 h after transfection. Firefly and Renilla luciferase activities were measured using the Dual-Luciferase Reporter Assay System (Promega). Light emission was measured with a Lumat LB9501 (Berthold) luminometer. GDNF protein concentrations in cell supernatants were determined using an ELISA kit (Promega). Firefly luciferase activity and GDNF concentration were normalized to the Renilla luciferase activity in the cell extract. For each line, three to five independent transfection experiments were performed.

### RESULTS

### 1. Effects of WPRE, tau 3'UTR, TH 3'UTR and APP 5'UTR on luciferase expression

Ability of WPRE, tau 3'UTR, TH 3'UTR and APP 5'UTR was tested to enhance luciferase expression in neural cells. In reporter plasmids each of the four elements was placed either in the 3' or 5' end of the firefly luciferase reporter gene (Fig. 1A). All the constructions were made in the pGL3-Control vector, which was used as a control plasmid. WPRE, tau3'UTR and TH3'UTR were inserted between the stop codon of the luciferase and the SV40 polyadenylation signal (pluc-WPRE, pluc-tau and pluc-TH). APP 5'UTR was placed between the SV40 promoter and the Kozak sequence (pAPP-luc). Luciferase expression from each vector was analysed by transient transfections of cell lines of neuronal (SKNSH and PC12 both treated and not treated with NGF to enhance neuronal differentiation) and glial (U-87M and C6) phenotypes. Both human cell lines (SKNSH, U-87MG) and rat cell lines (PC12, C6) were used because rats are widely used for developing CNS gene therapy protocols. Luciferase expression from pGL3-Control vector was assigned as 1.0 and activity of all other vectors is expressed relative to this value.

In both neuronal and glial cell lines, the highest levels of luciferase expression were obtained with pluc-WPRE (Fig. 1B, a and b). It increased expression by 4-fold in NGF-treated PC12, and 7-fold in PC12 and SKNSH (Fig. 1B, a). In glial cells, the level of stimulation by WPRE was also significant but lower to those observed in neuronal cell lines: luciferase expression was 4.5-fold the control value in C6 and 2.6-fold in U-87MG (Fig. 1B , b). APP 5'UTR also increased luciferase expression in both neuronal and glial cell lines. APP 5'UTR yielded over 1.9 to 2.5-fold higher expression in neuronal cells and 1.6 to 2.0-fold higher expression in glial cells. In both glial cell lines, C6 and U-87MG, tau 3'UTR and TH 3'UTR failed to increase luciferase expression significantly. In NGF-treated PC12 and SKNSH, tau 3'UTR and TH 3'UTR doubled expression (1.8 and 1.6 for tau 3'UTR, 2.0 and 1.9 with TH 3'UTR). In PC12, tau 3'UTR and TH 3'UTR increased expression 1.5-fold and 1.4-fold, respectively.

Thus luciferase expression was significantly enhanced by WPRE, APP 5'UTR, tau 3'UTR and TH 3'UTR in neuronal cell lines and by WPRE or APP 5'UTR in glial cell lines.

### 2. The effects of WPRE in combination with either tau 3'UTR, TH 3'UTR, or APP 5'UTR.

Combinations of the posttranscriptional regulatory elements were tested. As WPRE had the strongest capability of promoting luciferase expression in both neuronal and glial cells, said WPRE was tested with each of the other elements (tau 3'UTR, TH 3'UTR and APP 5'UTR). A series of vectors combining pairs of elements were obtained by inserting the WPRE downstream from the luciferase gene in pluc-tau, pluc-TH and pAPP-luc (Fig. 2A).

All combinations of two elements yielded an expression level similar to that with WPRE in both neuronal and glial cells (data not shown). When one-tenth the amount of plasmid (58 fmoles instead of 580 fmoles) was used to transfect cells these combinations of elements substantially increased luciferase expression (Fig. 2B, a). In PC12, expression was enhanced by 17, 13.5 or 14-fold when WPRE was associated with tau 3'UTR, TH 3'UTR or APP 5'UTR, respectively. These combinations resulted in 5-fold, 6.5-fold and 8-fold enhancements in NGF-treated PC12 and in 11-, 11-, 13-fold enhancements in SKNSH. Similarly, in glial cell lines, only the combination of WPRE with APP 5'UTR increased luciferase expression (Fig. 2B, b): in C6 and U-87MG, expression was enhanced by factors of 7 and 4, respectively. As expected no further increase was detected when WPRE was combined with tau 3'UTR or TH 3'UTR in these cells. The constructs pluc-tau-WPRE and pluc-TH-WPRE yielded lower luciferase expression than pluc-WPRE in both C6 and U-87MG. These data confirm the authors observation that the ability of tau 3'UTR and TH 3'UTR to promote luciferase expression is restricted to neuronal cell types.

Figure 7 shows the combined effects of WPRE and tau, of WPRE and TH 3'UTR, of WPRE and APP 5'UTR, relative to that of the individual elements (Figure 7, compare lines 1,2,3 and 7 with lines 8,9 and 10). For example, in PC12, the 14.5-fold enhancement observed with combination of WPRE and APP 5'UTR is equivalent to the product of the increases recorded for WPRE alone and APP 5'UTR alone (6.9-fold and 1.9-fold, respectively). Thus WPRE acts in synergy with the eucaryotic elements on expression in neuronal and glial cell lines.

### 3. The effects of other combinations of WPRE, tau 3'UTR, TH 3'UTR and APP 5'UTR in neuronal cell lines.

Combinations of tau 3'UTR, TH 3'UTR and APP 5'UTR were tested. A series of vectors that associate two eucaryotic element (pAPP-luc-tau and pAPP-luc-TH) or the three elements (pAPP-luc-TH-tau) were constructed in pAPP-luc (Fig. 3A). In neuronal cell lines, combinations of tau 3'UTR, TH 3'UTR and APP 5'UTR resulted in higher luciferase expression than APP 5'UTR alone (Fig. 3B). In PC12, pAPP-luc-tau, pAPP-luc-TH and pAPP-luc-TH-tau enhanced expression 2.7-fold, 2.4-fold and 3.4-fold, respectively. In NGF-treated PC12, the corresponding results were 3.6-fold, 2.9-fold and 5.4-fold enhancements. In SKNSH, the enhancements were 3.5-fold, 3.7-fold and 3.7-fold. Thus, combinations of tau 3'UTR, TH 3'UTR and APP 5'UTR synergistically increased luciferase expression in PC12 and NGF-treated PC12 (Figure 7, compare lines 1, 2 and 3 with lines 4, 5 and 6). In SKNSH, APP 5'UTR associated with tau 3'UTR or with TH 3'UTR synergistically increased luciferase expression, but combination of all three elements yielded an expression level similar to that with only two elements. This suggests that tau 3'UTR and TH 3'UTR have no cumulative effect in SKNSH.

Finally, the effectiveness of the combination of the four posttranscriptional elements were investigated in PC12, NGF-treated PC12 and also the effectiveness of the combination of WPRE with tau 3'UTR and APP 5'UTR in SKNSH. WPRE was inserted into the vectors pAPP-luc-TH-tau and pAPP-luc-tau upstream from the tau 3'UTR to obtain pAPP-luc-TH-WPRE-tau and pAPP-luc-WPRE-tau, respectively (Fig. 4A). These combinations resulted in a very substantial increase in luciferase expression: in PC12 and NGF-treated PC12, in the presence of all four elements the luciferase expression was over 26 and 25 times baseline, respectively (Fig. 4B). In SKNSH, the combination of WPRE, tau 3'UTR and APP 5'UTR resulted in a 17-fold enhancement (Fig. 4B). The levels of enhancement obtained with combination of WPRE, tau 3'UTR, Figure 7, compare lines 1, 2, 3, and 7 with lines 11 for SKNSH and 12 for PC12 and NGF-treated PC12).

### 4. Vector dose-dependence of enhancement of expression by WPRE, tau 3'UTR, TH 3'UTR and APP 5'UTR.

To determine which of these elements was affected by copy number, we transfected PC12 cells were transfected with various amounts (2, 4, 6, 8 and 16 µg) of pluc-WPRE, pluc-tau, pluc-TH and pAPP-luc. Luciferase expression was determined and compared to that obtained with pGL3-Control (defined as 1.0) for each dose of plasmid. The fact that luciferase expression increased linearly with the dose of plasmid was first verified to confirm that transcription was not limiting over the range tested (data not shown). All the four elements were affected by the number of plasmids transfected: increasing the amount of plasmid dose-dependently decreased the effect of tau 3'UTR, TH 3'UTR, APP 5'UTR and WPRE pluc-WPRE on expression (Figure 5).

Regulation of luciferase expression by WPRE, tau 3'UTR, TH 3'UTR and APP 5'UTR was thus affected by the number of plasmid molecules used for transfection.

### 5. The combination of WPRE and APP 5'UTR and that of WPRE and tau 3'UTR and APP 5'UTR enhanced expression of the GDNF gene.

The effects of WPRE and the eukaryotic elements and combination between them with the GDNF gene, a candidate for therapeutic purposes were tested. It was first verified whether WPRE, tau 3'UTR, TH 3'UTR and APP 5'UTR affected GDNF gene expression. pgdnf-WPRE, pgdnf-tau, pgdnf-TH and pgdnf-APP (Fig.6A) were then constructed. GDNF secreted into the culture supernatant from neuronal and glial cell lines transfected with these constructs was quantified by ELISA and compared to that obtained for pgdnf. GDNF expression was enhanced by tau 3'UTR, APP 5'UTR and WPRE in PC12 and NGF-treated PC12. In SKNSH and glial cells GDNF expression was increased with WPRE and APP 5'UTR (Fig.6B). TH 3'UTR did not significantly enhance GDNF secretion, it thus was not used in the subsequent constructions. The results were similar to those with the luciferase gene, indicating that activity of these elements is not specific to the transgene. WPRE combined with APP 5'UTR in SKNSH, glial cells (C6, U-87MG) and with APP 5'UTR and tau 3'UTR were then tested in neuronal cells (PC12, NGF-treated PC12). In glial cells, the combination of WPRE and APP 5'UTR resulted in a 10- and 2-fold higher expression than that from pgdnf and pgdnf-WPRE in C6 and 5- and 1.4-fold higher expression than that from pgdnf and pgdnf-WPRE in U-87MG (Fig.6B). In SKNSH, this combination resulted in a 3.7 and 1.4-fold higher expression. In PC12 and NGF-treated PC12, pAPP-gdnf-WPRE-tau yielded 15- and 7-fold higher expression than pgdnf, respectively and about 3-fold higher expression than pgdnf-WPRE (Fig.6B). The enhancement of expression obtained with the combination of WPRE, tau 3'UTR and APP 5'UTR in neuronal cells and with the combination of WPRE and APP 5'UTR in glial cells indicated that the effects of these elements on GDNF expression were synergistic.

### DISCUSSION

The invention now allows the use of posttranscriptional regulatory elements for high-level gene expression in the CNS. The rationale for using neural cell-specific DNA regulatory elements is that target CNS cells contain neural trans-acting factors that normally interact with these elements. The only elements which were supposed to increase efficiency of steps of the posttranscriptional pathway in neuronal and/or astrocyte cells were WPRE and sequences from rat tau 3'UTR, rat TH 3'UTR and human APP 5'UTR. These elements increase expression in rat neuronal cell lines (TH 3'UTR, Paulding and Czyzyk-Krzeska, 1999) or in both human and rat neuronal cell lines (tau 3'UTR, Aronov *et al*, 1999); in neurons *in vivo* (WPRE, Kaplitt *et al*, 1994) or in both neuronal and glial human cell lines (5'UTR APP, Rogers *et al*, 1999). These previous observations were confirmed and the present invention now further demonstrate that WPRE improves gene expression in neuronal as well as glial cells and that TH 3'UTR and APP 5'UTR improve expression in cells from both rats and humans. All four sequences are thus active in both rat and human cells. The invention also demonstrate that these elements have synergistic effects on gene expression.

Among the four posttranscriptional regulatory elements tested, WPRE have the largest effect on transgene expression in neural cell lines of both human and rat origin. It substantially enhances expression, especially in neuronal cells. These high levels of enhancement are similar with those observed in other transfected and infected cultured cell types (Zufferey *et al*, 1999; Loeb *et al*, 1999). Effects of tau 3'UTR, TH 3'UTR and APP 5'UTR on gene expression are not as pronounced. APP 5'UTR doubles expression in both neuronal and glial cells whereas tau 3'UTR and TH 3'UTR also double expression but only in neuronal cells. Unlike WPRE, that evolved to ensure high level of expression of viral genes by cumulating different functions, posttranscriptional regulatory elements from tau 3'UTR, TH 3'UTR and APP 5'UTR are involved in the regulation of expression of specific genes. The effects of these sequences are moderate and specific to the cell type. The sequence from tau 3'UTR stabilizes tau mRNA and ensures that it is localized near the microtubules during neuronal differentiation. This process is mediated by the neuron-specific ELAV-like HuD RNA-binding protein (Aranda-Abreu *et al*, 1999). The expression of this protein only in neurons explains the neurospecificity of the tau 3'UTR effect. The level of this protein is increased in PC12 on NGF-induced differentiation (Dobashi *et al*, 1998), which correlates well with the increased expression enhancement by tau 3'UTR observed on neuronal differentiation. TH 3'UTR contains a 28-base sequence that is a stabilizing element necessary for both constitutive and hypoxia-regulated stability of TH mRNA. This sequence is recognized by poly(C)-binding protein, PCBP (Holcik and Liebhaber, 1997; Paulding and Czyzyk-Krzeska, 1999). Since PCBP appears to be ubiquitous (Aasheim *et al*, 1994; Leffers *et al*, 1995) and TH 3'UTR stimulates expression only in neuronal cells it will further be important to determine how PCBP is regulated to understand the mechanism of stimulation by TH 3'UTR.

The four posttranscriptional regulatory elements tau 3'UTR, TH 3'UTR, APP 5'UTR and WPRE have synergistic effects on expression when they are combined. This synergy suggests that the functions of these elements are not redundant. Presumably WPRE, APP 5'UTR, tau 3'UTR and TH 3'UTR improve the efficiency of different steps in the posttranscriptional pathway or act at the same step but with different mechanisms. It has been shown previously that different steps are affected by these sequences. APP 5'UTR regulates mRNA translation (Rogers *et al*, 1999). The sequences from tau 3'UTR and TH 3'UTR act on mRNA stability (Aronov *et al*, 1999; Paulding and Czyzyk-Krzeska, 1999). WPRE acts at multiple steps but principally very early during the biogenesis of RNA transcripts, perhaps by directing their efficient processing as soon as they emerge from the transcriptional machinery (Zufferey *et al*, 1999; Loeb, 2000). To explain the synergy between elements that act at the same step, such as tau 3'UTR and TH 3'UTR on mRNA stability in PC12 cells, the molecular mechanisms by which these sequences affect gene expression need to be identified. It may reveal conditions required for synergism between tau 3'UTR and TH 3'UTR on expression, conditions that are present in PC12 cells both treated and untreated with NGF but not in SKNSH.

Posttranscriptional regulation may become rate limiting. The invention shows that the effects of WPRE, APP 5'UTR, tau 3'UTR and TH 3'UTR depend on the number of copies of the transgenes: the effects of the four elements on expression decreased with increasing dose of plasmid transfected. This suggests a titration of trans-acting factors by their target sequences and the fact that posttranscriptional processes mediated by these elements become rate limiting. This is presumably due to either low abundance of trans-acting factors in cells or high numbers of copies of mRNA transcribed by the strong SV40 promoter or both. It is interesting to note that in transient transfections, a fraction of the cells takes up a large proportion of the transfecting molecules.

Invention shows that inclusion of combinations between WPRE and APP 5'UTR, tau 3'UTR in vectors could be of benefit for CNS gene therapy. First, substantial enhancement of already efficiently expressed transgenes was obtained with these combinations. The highest level of expression in glial cells was obtained with APP 5'UTR plus WPRE and in neuronal cells with tau 3'UTR plus APP 5'UTR plus WPRE. These combinations allow therapeutic goals to be attained with substantially fewer vectors, thereby decreasing both the side effects associated with viral injection and the number of copies of transgenes required per cell. Second, these elements improve transgene expression in the two major cell target types for gene therapy of diseases affecting the CNS: neuronal and glial cells. Finally, the present invention shows that these combinations are also active with a therapeutic gene, GDNF. GDNF is a potent trophic factor for CNS dopamine-containing neurons (Bilang-bleuel *et al*, 1997; Kordower *et al*, 2000) and a potential candidate for the treatment of Parkinson disease.

In conclusion, the present invention demonstrates that improvement of posttranscriptionnal processes with the cis-acting elements tau 3'UTR, TH 3'UTR, APP 5'UTR and WPRE can further increase expression of an already well expressed transgene provided that mRNA concentrations in expressing cells are not too high, which would interfere with its regulated metabolism. Optimal combinations for high-level expression are association between WPRE and APP 5'UTR in glial cells and association between WPRE and tau 3'UTR and APP 5'UTR in neuronal cells. The results obtained by the authors of the invention provide important information regarding the construction of vectors in not only gene therapy but in the study of gene function as well.

### REFERENCES

AASHEIM, H.-C., LOUKIANOVA, T., DEGGERDAL, A., and SMELAND, E.B. (1994). Tissue specific expression and cDNA structure of a human transcript encoding a nucleic acid binding [oligo(dC)] protein related to the pre-mRNA binding protein K. Nucleic Acids Res. 22,959-964.
ANTONIOU, M., GERAGHTY, F., HURST J., and GROSVELD, F. (1998). Efficient 3'-end formation of human beta-globin mRNA in vivo requires sequences within the last intron but occurs independently of the splicing reaction. Nuclear Acids Res. 26, 721-729.
ARANDA-ABREU, G.E., BEHAR, L., CHUNG, S., FURNEAUX, H., and GINZBURG, I. (1999). Embryonic lethal abnormal vision-like RNA-binding proteins regulate neurite outgrowth and tau exprsssionin PC12 cells. J. Mol. Neurosci. 12, 1-15.
ARONOV S., MARX, R., and GINZBURG, I. (1999). Identification of 3'UTR region implicated in tau mRNA stabilization in neuronal cells. J. Mol. Neurosci. 12, 131-145.
BILANG-BLEUEL, A., REVAH, F., COLIN, P., LOCQUET, I., ROBERT, J.J., MALLET, J., and HORELLOU, P. (1997). Intrastriatal injection of an adenoviral vector expressing glial-cell-line-derived neurotrophic factor prevents dopaminergic neuron degeneration and behavioral impairment in a rat model of Parkinson disease. Proc. Natl. Acad. Sci. U.S.A. 16, 8818-8823
BUCHMAN, A.R., and BERG, P. (1988). Comparison of intron-dependent and intron-independent gene expression. Mol. Cell. Biol. 8, 4395-4405.
CHOI, T., HUANG, M., GORMAN, C., and JAENISCH, R. (1991). A generic intron increases gene expression in transgenic mice. Mol. Cell. Biol. 9, 3070-3074.
DOBASHI, Y., MITSUHIKO, S., WAKATA, Y., and KAMEYA, T. (1998). Expression of HuD protein is essential for initial phase of neuronal differentiation in PC12 cells. Biochem. Biophys. Res. Commun. 224, 226-229
DONELLO, J.E., LOEB, J.E., and HOPE, J.T. (1998). Woodchuck hepatitis virus contains a tripartite posttranscriptional regulatory element. J. Virol. 72, 5085-5092.
GRIMA, B., LAMOUROUX, A., BLANOT, F., FAUCON-BIGUET, N., and MALLET, J. (1985). Complete sequence of rat tyrosine hydroxylase mRNA. Proc. Natl. Acad. Sci. U.S.A. 82, 617-621.
HOLCIK, M., and LIEBHABER, S.A. (1997). Four highly stable eukaryotic mRNAs assemble 3' untranslated region RNA-protein complexes sharing cis and trans components. Proc. Natl. Acad. Sci. U.S.A. 94, 2410-2414.
HUANG, M.T., and GORMAN, C.M. (1990). Intervening sequences increase efficiency of RNA 3' processing and accumulation of cytoplasmic RNA. Nuclear Acids Res. 18, 937-947.
HUANG, Z.M., and YEN, T.S. (1995). Role of the hepatitis B virus posttranscriptional regulatory element in export of intronless transcripts. Mol. Cell. Biol. 15, 3864-3869.
HUANG, Y., WIMLER, K.M., and CARMICHAEL, G.G. (1999). Intronless mRNA transport elements may affect multiple steps of pre-mRNA processing. EMBO J. 18, 1642-1652.
KAPLITT, M.G., LEONE, P., SAMULSKI, R.J., XIAO, X., PFAFF, D.W., O'MALLEY, K.L., and DURING M.J. (1994). Long-term gene expression and phenotypic correction using adeno-associated virus vectors in the mammalian brain. Nat. Genet. 2, 148-154.
KORDOWER, J.H., EMBORG, M.E., BLOCH, J., MA, S.Y., CHU, Y., LEVENTHAL, L., MCBRIDE, J., CHEN, E.Y., PALFI, S., ROITBERG, B.Z., BROWN, W.D., HOLDEN, J.E., PYZALSKI, R., TAYLOR, M.D., CARVEY, P., LING, Z., TRONO, D., HANTRAYE, P., DEGLON, N., and AEBISCHER, P. (2000). Neurodegeneration prevented by lentiviral vector delivery of GDNF in primate models of Parkinson's disease. Science 5492, 767-773.
LEFFERS, H., DEJGAARD, K., and CELIS, J.E. (1995). Characterisation of two major cellular poly(rC)-binding human proteins, each containing three K-homologous (KH) domains. Eur. J. Biochem. 230, 447-453.
LE GAL LA SALLE, G., ROBERT, J.J., BERRARD, S., RIDOUX, V., STRATFORD-PERRICAUDET, L.D., PERRICAUDET, M., and MALLET J. (1993). An adenovirus vector for gene transfer into neurons and glia in the brain. Science 259, 988-990.
LOEB, J.E., CORDIER, W.S., HARRIS, M.E., WEITZMAN, M.D., and HOPE, T.J. (1999). Enhanced expression of transgenes from adeno-associated virus vectors with the woodchuck hepatitis virus posttranscriptional regulatory element: implications for gene therapy. Hum. Gene. Ther. 10, 2295-2305.
LOEB, J., HARRIS, M., and HOPE, T. (2000). The woodchuck hepatitisvirus posttranscriptional regulatory element increases transgene expression by enhancing the 3'-end metabolism of mRNAs. Mol. Ther. 1, S142.
NALDINI, L., BLOMER, U., GALLAY, P., ORY, D., MULLIGAN, R., GAGE, F.H., VERMA, I.M., and TRONO, D. (1996). In vivo gene delivery and stable transduction of nondividing cells by a lentiviral vector. 272, 263-267
NESIC, D., CHENG, J., and MAQUAT L.E. (1993). Sequences within the last intron function in RNA 3'-end formation in cultured cells. Mol. Cell. Biol. 13, 3359-3369.
PAULDING, W.R., and CZYZYK-KRZESKA, M.F. (1999). Regulation of tyrosine hydroxylase mRNA stability by protein binding, pyrimidine-rich sequence in the 3'-untranslated region. J. Biol. Chem. 274, 2532-2538.
RAMEZANI, A., HAWLEY, T.S., and HAWLEY, R.G. (2000). Lentiviral vectors for enhanced gene expression in human hematopoietic cells. Mol. Ther. 2, 458-469.
ROGERS, J.T., LEITER, L.M., MCPHEE J., CAHILL, C.M., ZHAN, S.-S., POTTER H., and NILSSON, L.N.G. (1999). Translation of the Alzheimer Amyloid precursor protein mRNA is up-regulated by interleukin-1 through 5'-untranslated region sequences. J. Biol. Chem. 274, 6421-6431.
RYU, W.-S., and MERTZ, J.E. (1989). Simian virus 40 late transcripts lacking excisable intervening sequences are defective in both stability in the nucleus and transport to the cytoplasm. J. Virol. 63, 4386-4394.
SCHAMBACH, A., WODRICH, H., HILDINGER, M., BOHNE, J., KRAUSSLICH, H.-G., and BAUM, C. (2000). Context dependence of different modules for posttranscriptional enhancement of gene expression from retroviral vectors. MoLTher. 2, 435-4445.
THOMAS, C.E., BIRKETT, D., ANOZIE, I., CASTRO, M.G., and LOWENSTEIN, P.R. (2001). Acute direct adenoviral vector cytotoxicity and chronic, but not acute, inflammatory responses correlate with decreased vector-mediated transgene expression in the brain. Mol. Ther. 3, 36-46.
ZUFFEREY, R, DONELLO, J.E., TRONO D., and HOPE T.J. (1999). Woodchuck hepatitis virus posttranscriptional regulatory element enhances expression of transgenes delivered by retroviral vectors. J. Virol. 73, 2886-2892.

## Claims

1. A vector suitable for transgene delivery into mammalian neuronal cells, wherein said vector comprises a chimeric genetic construct comprising a transgene operably linked to at least two distinct posttranscriptional regulatory elements functional in cells, one of said posttranscriptional regulatory elements being the tau 3'UTR region of a eukaryotic mRNA or a functional portion thereof, and the other one being all or a functional portion of a WPRE element.

2. The vector according to claim 1, wherein said vector comprises a chimeric genetic construct comprising a transgene operably linked to a WPRE element, an APP5'UTR region and a tau3'UTR region.

3. The vector according to claim 2, wherein said vector comprises a chimeric genetic construct comprising a transgene operably linked to a WPRE element, an APP5'UTR region, a tau3'UTR region and a TH3'UTR region.

4. The vector of any one of claims 1 to 3, wherein said WPRE element comprises all or a functional fragment of SEQ ID NO: 1.

5. The vector of any one of claims 2 or 3, wherein said APP5'UTR region comprises all or a functional fragment of SEQ ID NO: 2.

6. The vector of any one of claims 1 to 5, wherein said tau3'UTR region comprises all or a functional fragment of SEQ ID NO: 3.

7. The vector of claim 3 or 4, wherein said TH3'UTR region comprises all or a functional fragment of SEQ ID NO: 4.

8. The vector of any one of the preceding claims, wherein said vector further comprises a promoter controlling transcription of the transgene in said mammalian neuronal cells.

9. The vector of any one of the preceding claims, wherein said vector further comprises a marker gene.

10. The vector of any one of the preceding claims, wherein said vector further comprises a polyadenylation signal operably linked to said transgene.

11. The vector of any one of the preceding claims, wherein said vector is selected from a plasmid and a recombinant virus.

12. The vector of claim 11, wherein said vector is a replication-defective adenovirus, a replication-defective adeno-associated virus or a replication-defective retrovirus, including replication-defective lentiviruses.

13. The vector of any one of the preceding claims, wherein the transgene is selected from a transgene coding for a growth factor, a neurotrophic factor, a cytokine, a ligand, a receptor, an immunoglobulin and an enzyme.

14. A recombinant cell comprising a chimeric genetic construct as described in anyone of claims 1 to 13 or a vector of any one of claims 1 to 13.

15. Use of a vector of anyone of claims 1 to 13 or a recombinant cell of claim 14 for the manufacture of a medicament to treat a human neurodegenerative disease.

16. A composition comprising a chimeric genetic construct as described in anyone of claims 1 to 13, a vector of anyone of claims 1 to 13 or a recombinant cell of claim 14 and a pharmaceutically acceptable excipient or carrier.

17. The composition of claim 16 for treating a human neurodegenerative disease.

18. The use of claim 15 or the composition of claim 17, wherein the neurodegenerative disease is selected from Parkinson disease, Alzheimer's disease, amyotrophic lateral sclerosis (ALS), Huntington's disease and retinal degenerative diseases.

19. A vector of anyone of claims 1 to 13 or a recombinant cell of claim 14 for treating a human neurodegenerative disease.

20. A method of expressing a transgene in a mammalian neuronal cell *in vitro* or *ex vivo*, the method comprising:
a. providing a chimeric genetic construct comprising said transgene operably linked to at least two distinct posttranscriptional regulatory elements, one of said posttranscriptional regulatory elements being the tau 3'UTR region of a eukaryotic mRNA or a functional portion thereof, and the other one being all or a functional portion of a WPRE element, and
b. introducing said construct into mammalian neuronal cells, said introduction causing expression of said transgene in said mammalian neuronal cells.

21. The method of claim 20, comprising:
a. providing a vector according to any one of claims 1-13, and
b. introducing said vector into mammalian neuronal cells, said introduction causing expression of said transgene in said mammalian neuronal cells.

22. The method of claim 21, wherein said mammalian neuronal cell is a human neuronal cell or a rodent neuronal cell.

23. The method of any one of claims 20 to 22, wherein the chimeric genetic construct or vector is introduced into said mammalian neuronal cells by virus-mediated infection.

24. The method of any one of claims 20 to 22, wherein the chimeric genetic construct or vector is introduced into said cells by plasmid-mediated transfection.

25. An in vitro method of expressing a transgene in neuronal cells, the method comprising:
a. providing a chimeric genetic construct comprising said transgene operably linked to posttranscriptional regulatory elements comprising a WPRE element combined with a APP5'UTR and a tau3'UTR or a portion thereof, and
b. introducing said construct into neuronal cells, said introduction causing expression of said transgene in said neuronal cells.

26. An in vitro method of expressing a transgene in neuronal cells, the method comprising:
a. providing a chimeric genetic construct comprising said transgene operably linked to posttranscriptional regulatory elements comprising a WPRE element combined with a APP5'UTR, a tau3'UTR and a TH3'UTR or a portion thereof,
b. introducing said construct into neuronal cells, said introduction causing expression of said transgene in said neuronal cells.

## Patentansprüche

1. Ein Vektor geeignet für die Zufuhr von einem Transgen in neuronale Säugetierzellen, wobei der Vektor ein chimäres genetisches Konstrukt umfasst, das ein Transgen operabel mit mindestens zwei unterschiedlichen posttranskriptionalen regulatorischen Elementen verknüpft umfasst, wobei die posttranskriptionalnen regulatorischen Elemente funktional in Zellen sind, und wobei einer dieser posttranskriptionalen regulatorischen Elemente die tau 3'UTR Region einer eukaryotischen mRNA oder ein funktioneller Teil davon ist, und der andere ein komplettes oder ein funktionaler Teil eines WPRE Elementes ist.

2. Der Vektor nach Anspruch 1, wobei der Vektor ein chimäres genetisches Konstrukt umfasst, das ein Transgen operabel mit einem WPRE Element, einer APP5'UTR Region und einer tau3'UTR Region verknüpft umfasst.

3. Der Vektor nach Anspruch 2, wobei der Vektor ein chimäres genetisches Konstrukt umfasst, das ein Transgen operabel mit einem WPRE Element, einer APP5'UTR Region, einer tau3'UTR Region und einer TH3'UTR Region verknüpft umfasst.

4. Der Vektor nach einem der Ansprüche 1 bis 3, wobei das WPRE Element die SEQ ID NO: 1 oder ein funktionelles Fragment davon umfasst.

5. Der Vektor nach einem der Ansprüche 2 oder 3, wobei die APP5'UTR Region die SEQ ID NO: 2 oder ein funktionelles Fragment davon umfasst.

6. Der Vektor nach einem der Ansprüche 1 bis 5, wobei die tau3'UTR Region die SEQ ID NO: 3 oder ein funktionelles Fragment davon umfasst.

7. Der Vektor nach Anspruch 3 oder 4, wobei die TH3'UTR Region die SEQ ID NO: 4 oder ein funktionelles Fragment davon umfasst.

8. Der Vektor nach einem der vorhergehenden Ansprüche, wobei der Vektor ferner einen Promotor umfasst, der die Transkription des Transgens in der neuronalen Säugetierzelle reguliert.

9. Der Vektor nach einem der vorhergehenden Ansprüche, wobei der Vektor ferner ein Markergen umfasst.

10. Der Vektor nach einem der vorhergehenden Ansprüche, wobei der Vektor ferner ein Polyadenylierungs-Signal umfasst, das operabel mit dem Transgen verknüpft ist.

11. Der Vektor nach einem der vorhergehenden Ansprüche, wobei der Vektor ausgewählt ist aus einem Plasmid und einem rekombinanten Virus.

12. Der Vektor nach Anspruch 11, wobei der Vektor ein replikationsdefizienter Adenovirus, ein replikationsdefizienter Adeno-assozüerter Virus oder ein replikationsdefizienter Retrovirus einschließlich eines replikationsdefizienten Lentivirus ist.

13. Der Vektor nach einem der vorhergehenden Ansprüche, wobei das Transgen ausgewählt ist aus einem Transgen kodierend einen Wachstumsfaktor, einen neurotrophischen Faktor, ein Cytokin, einen Liganden, einen Rezeptor, ein Immunoglobulin und ein Enzym.

14. Eine rekombinante Zelle umfassend ein chimäres genetisches Konstrukt wie in einem der Ansprüche 1 bis 13 beschrieben oder einen Vektor nach einem der Ansprüche 1 bis 13.

15. Verwendung eines Vektors nach einem der Ansprüche 1 bis 13 oder einer rekombinanten Zelle nach Anspruch 14 für die Herstellung eines Medikamentes zur Behandlung einer menschlichen neurogenerativen Erkrankung.

16. Eine Zusammensetzung umfassend ein chimäres genetisches Konstrukt wie in einem der Ansprüche 1 bis 13 beschrieben, einen Vektor nach einem der Ansprüche 1 bis 13 oder eine rekombinante Zelle nach Anspruch 14 und einen pharmazeutisch verträglichen Hilfsstoff oder Träger.

17. Die Zusammensetzung nach Anspruch 16 zur Behandlung einer humanen neurogenerativen Erkrankung.

18. Die Verwendung nach Anspruch 15 oder die Zusammensetzung nach Anspruch 17, wobei die neurogenerative Erkrankung ausgewählt ist aus der Parkinson-Krankheit, der Alzheimer-Krankheit, der Amyotrophen Lateralsklerose (ALS), der Huntington Krankheit und einer degenerative Erkrankung der Retina.

19. Ein Vektor nach einem der Ansprüche 1 bis 13 oder eine rekombinante Zelle nach Anspruch 14 zur Behandlung einer humanen neurodegenerativen Erkrankung.

20. Ein Verfahren zur Expression eines Transgens in einer neuronalen Säugetierzelle *in vitro* oder *ex vivo,* wobei das Verfahren die folgenden Schritte umfasst:
a. zur Verfügungstellen eines chimären genetischen Konstruktes umfassend das Transgen operabel mit mindestens zwei unterschiedlichen posttranskriptionalen regulatorischen Elementen verknüpft, wobei einer dieser posttranskriptionalen regulatorischen Elemente die tau 3'UTR Region einer eukaryotischen mRNA oder ein funktioneller Teil davon ist, und der andere ein komplettes oder ein funktionaler Teil eines WPRE Elementes ist, und
b. Einbringen des Konstruktes in neuronale Säugetierzellen, wobei das Einbringen zur Expression des Transgens in den neuronalen Säugetierzellen führt.

21. Das Verfahren nach Anspruch 20, umfassend:
a. zur Verfügungstellen eines Vektors nach einem der Ansprüche 1 bis 13, und
b. Einbringen des Vektors in neuronale Säugetierzellen, wobei das Einbringen zur Expression des Transgens in den neuronalen Säugetierzellen führt.

22. Das Verfahren nach Anspruch 21, wobei die neuronale Säugetierzelle eine humane neuronale Zelle oder eine neuronale Nagetierzelle ist.

23. Das Verfahren nach einem der Ansprüche 20 bis 22, wobei das chimäre genetische Konstrukt oder der Vektor durch Virus-vermittelte Infektion in die neuronalen Säugetierzellen eingebracht wird.

24. Das Verfahren nach einem der Ansprüche 20 bis 22, wobei das chimäre genetische Konstrukt oder der Vektor durch Plasmid-vermittelte Transfektion in die Zellen eingebracht wird.

25. Ein *in vitro* Verfahren zur Expression eines Transgens in neuronalen Zellen, wobei das Verfahren die folgenden Schritte umfasst:
a. zur Verfügungstellen eines chimären genetischen Konstruktes umfassend das Transgen operabel verknüpft mit posttranskriptionalen regulatorischen Elementen, wobei die posttranskriptionalen regulatorischen Elemente ein WPRE Element kombiniert mit einem APP5'UTR und einem tau3'UTR oder einem Teil davon umfassen, und
b. Einbringen des Konstruktes in neuronale Zellen, wobei das Einbringen zur Expression des Transgens in den neuronalen Zellen führt

26. Ein *in vitro* Verfahren zur Expression eines Transgens in neuronalen Zellen, wobei das Verfahren die folgenden Schritte umfasst:
a. zur Verfügungstellen eines chimären genetischen Konstruktes umfassend das Transgen operabel verknüpft mit posttranskriptionalen regulatorischen Elementen, wobei die posttranskriptionalen regulatorischen Elemente ein WPRE Element kombiniert mit einem APP5'UTR, einem tau3'UTR und einem TH3'UTR oder einem Teil davon umfassen,
b. Einbringen des Konstruktes in neuronale Zellen, wobei das Einbringen zur Expression des Transgens in den neuronalen Zellen führt.

## Revendications

1. Vecteur adapté à la délivrance d'un transgène dans des cellules neuronales de mammifère, dans lequel ledit vecteur comprend un construit génétique chimérique comprenant un transgène lié de façon opérationnelle à au moins deux éléments de régulation post-transcriptionnels distincts fonctionnels dans des cellules, l'un desdits éléments de régulation post-transcriptionnels étant la région tau 3'UTR d'un ARNm eucaryote ou une portion fonctionnelle de celle-ci, et l'autre étant un élément WPRE ou une portion fonctionnelle d'un élément WPRE.

2. Le vecteur selon la revendication 1, dans lequel ledit vecteur comprend un construit génétique chimérique comprenant un transgène lié de façon opérationnelle à un élément WPRE, une région APP5'UTR et une région tau3'UTR.

3. Le vecteur selon la revendication 2, dans lequel ledit vecteur comprend un construit génétique chimérique comprenant un transgène lié de façon opérationnelle à un élément WPRE, une région APP5'UTR, une région tau3'UTR et une région TH3'UTR.

4. Le vecteur selon l'une quelconque des revendications 1 à 3, dans lequel ledit élément WPRE comprend SEQ ID NO : 1 ou un fragment fonctionnel de SEQ ID NO : 1.

5. Le vecteur selon l'une quelconque des revendications 2 ou 3, dans lequel ladite région APP5'UTR comprend SEQ ID NO :2 ou un fragment fonctionnel de SEQ ID NO :2.

6. Le vecteur selon l'une quelconque des revendications 1 à 5, dans lequel ladite région tau3'UTR comprend SEQ ID NO :3 ou un fragment fonctionnel de SEQ ID NO :3.

7. Le vecteur selon la revendication 3 ou 4, dans lequel ladite région TH3'UTR comprend SEQ ID NO :4 ou un fragment fonctionnel de SEQ ID NO :4.

8. Le vecteur selon l'une quelconque des revendications précédentes, dans lequel ledit vecteur comprend en outre un promoteur contrôlant la transcription du transgène dans lesdites cellules neuronales de mammifère.

9. Le vecteur selon l'une quelconque des revendications précédentes, dans lequel ledit vecteur comprend en outre un gène marqueur.

10. Le vecteur selon l'une quelconque des revendications précédentes, dans lequel ledit vecteur comprend en outre un signal de polyadénylation lié de façon opérationnelle audit transgène.

11. Le vecteur selon l'une quelconque des revendications précédentes, dans lequel ledit vecteur est choisi parmi un plasmide et un virus recombinant.

12. Le vecteur selon la revendication 11, dans lequel ledit vecteur est un adénovirus défectif pour la réplication, un virus adéno-associé défectif pour la réplication ou un rétrovirus défectif pour la réplication, incluant les lentivirus défectifs pour la réplication.

13. Le vecteur selon l'une quelconque des revendications précédentes, dans lequel le transgène est choisi parmi un transgène codant pour un facteur de croissance, un facteur neurotrophique, une cytokine, un ligand, un récepteur, une immunoglobuline et un enzyme.

14. Une cellule recombinante comprenant un construit génétique chimérique tel que décrit dans l'une quelconque des revendications 1 à 13 ou un vecteur selon l'une quelconque des revendications 1 à 13.

15. Utilisation d'un vecteur selon l'une quelconque des revendications 1 à 13 ou d'une cellule recombinante selon la revendication 14 pour la préparation d'un médicament pour traiter une maladie neurodégénérative humaine.

16. Une composition comprenant un construit génétique chimérique tel que décrit dans l'une quelconque des revendications 1 à 13, un vecteur selon l'une quelconque des revendications 1 à 13 ou une cellule recombinante selon la revendication 14 et un excipient ou un support acceptable sur le plan pharmaceutique.

17. La composition selon la revendication 16 pour traiter une maladie neurodégénérative humaine.

18. L'utilisation selon la revendication 15 ou la composition selon la revendication 17, dans laquelle la maladie neurodégénérative est choisie parmi la maladie de Parkinson, la maladie d'Alzheimer, la sclérose latérale amyotrophique (ALS), la maladie de Huntington et les maladies dégénératives de la rétine.

19. Un vecteur selon l'une quelconque des revendications 1 à 13 ou une cellule recombinante selon la revendication 14 pour traiter une maladie neurodégénérative humaine.

20. Une méthode d'expression d'un transgène dans une cellule neuronale de mammifère *in vitro* ou *ex vivo,* la méthode comprenant :
a. fournir un construit génétique chimérique comprenant ledit transgène lié de manière opérationnelle à au moins deux éléments de régulation post-transcriptionnels distincts, l'un desdits éléments de régulation post-transcriptionnels étant la région tau3'UTR d'un ARNm eucaryote ou une portion fonctionnelle de celle-ci, et l'autre étant un élément WPRE ou une portion fonctionnelle d'un élément WPRE, et
b. introduire ledit construit dans des cellules neuronales de mammifère, ladite introduction provoquant l'expression dudit transgène dans lesdites cellules neuronales de mammifère.

21. La méthode selon la revendication 20, comprenant :
a. fournir un vecteur selon l'une quelconque des revendications 1-13, et
b. introduire ledit vecteur dans des cellules neuronales de mammifère, ladite introduction provoquant l'expression dudit transgène dans lesdites cellules neuronales de mammifère.

22. La méthode selon la revendication 21, dans laquelle ladite cellule neuronale de mammifère est une cellule neuronale humaine ou une cellule neuronale de rongeur.

23. La méthode selon l'une quelconque des revendications 20 à 22, dans laquelle le construit génétique chimérique ou le vecteur est introduit dans lesdites cellules neuronales de mammifère par infection par l'intermédiaire d'un virus.

24. La méthode selon l'une quelconque des revendications 20 à 22, dans laquelle le construit génétique chimérique ou le vecteur est introduit dans lesdites cellules par transfection par l'intermédiaire d'un plasmide.

25. Une méthode d'expression *in vitro* d'un transgène dans des cellules neuronales, la méthode comprenant :
a. fournir un construit génétique chimérique comprenant ledit transgène lié de manière opérationnelle à des éléments de régulation post-transcriptionnels comprenant un élément WPRE combiné à un APP5'UTR et un tau3'UTR ou une portion de ceux-ci, et
b. introduire ledit construit dans des cellules neuronales, ladite introduction provoquant l'expression dudit transgène dans lesdites cellules neuronales.

26. Une méthode d'expression *in vitro* d'un transgène dans des cellules neuronales, la méthode comprenant :
a. fournir un construit génétique chimérique comprenant ledit transgène lié de manière opérationnelle à des éléments de régulation post-transcriptionnels comprenant un élément WPRE combiné à un APP5'UTR, un tau3'UTR et un TH3'UTR ou une portion de ceux-ci, et
b. introduire ledit construit dans des cellules neuronales, ladite introduction provoquant l'expression dudit transgène dans lesdites cellules neuronales.
